Europäisches Patentamt

European Patent Office

Office européen des brevets

(11) Publication number: **0 497 144 B1**

## EUROPEAN PATENT SPECIFICATION

(45) Date of publication of patent specification: **13.09.95**

(21) Application number: **92100590.6**

(22) Date of filing: **15.01.92**

The file contains technical information submitted after the application was filed and not included in this specification

(51) Int. Cl.⁶: **A61K 7/48**, A61K 7/02, A61K 7/021, A61K 7/031, A61K 7/42

(54) Styrene-ethylene-propylene copolymer containing cosmetic compositions and their use.

(30) Priority: **16.01.91 US 642196**

(43) Date of publication of application: **05.08.92 Bulletin 92/32**

(45) Publication of the grant of the patent: **13.09.95 Bulletin 95/37**

(84) Designated Contracting States: **DE FR GB IT**

(56) References cited:
**EP-A- 0 154 831**
**WO-A-88/01164**
**FR-A- 2 367 486**

**F. W. Billmeyer, Jr.; Textbook of Polymer Science, 3rd ed., John Wiley & Sons, Inc., (1984) pp 120-121, 336 and 347-351**

(73) Proprietor: **ESTEE LAUDER INC.**
**767 Fifth Avenue**
**New York**
**New York 10022 (US)**

(72) Inventor: **Chung, Kenneth K.**
**23 Manor Road**
**North Greenlawn,**
**New York 11740 (US)**
Inventor: **Nardolillo, Irene**
**15 Troy Court**
**Northport,**
**New York 11768 (US)**

(74) Representative: **VOSSIUS & PARTNER**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

This invention relates to styrene-ethylene-propylene copolymer containing cosmetic compositions and methods for using such compositions. The compositions of the invention have a number of desirable characteristics, including that they leave a smooth texture when applied to the skin, are easily spreadable, and are relatively transferproof.

Certain components that are sometimes used in cosmetic compositions (e.g., fatty soaps) can form an unappealing texture when applied to the skin. Cosmetic compositions that contain such components tend to flake-off from the skin, are difficult to apply, and frequently are water soluble. Cosmetic compositions that do not exhibit the foregoing characteristics are, of course, desirable.

Cosmetic compositions typically contain one or more colorants. Alternatively (or in addition), they may contain sunblock agents to provide the skin protection from the harmful effects of ultraviolet rays. In skin compositions that are intended for use in protecting the skin from the harmful effects of ultraviolet radiation, it is desirable that the sunblock agent be distributed uniformly throughout the composition.

An object of this invention is to provide a styrene-ethylene-propylene copolymer containing cosmetic composition that exhibits a combination of desirable properties, including that they leave a smooth texture when they are applied to the skin, they are readily spreadable on the skin, and they do not tend to flake-off the skin after application.

Another object of this invention is to provide a styrene-ethylene-propylene copolymer containing cosmetic composition for use as a sunblock.

Still another object of the invention is to provide an improved method for applying cosmetics (including cosmetics that contain a sunblock) to the skin.

The foregoing and other desirable advantages are obtained by a composition and the use of a composition that comprises:

(a) a first component comprising particulate styrene-ethylene-propylene copolymer, wherein the copolymer is a diblock copolymer having the structure S-EP, wherein "S" denotes a block comprising styrene monomers and "EP" denotes a block comprising ethylene and propylene monomers;

(b) a second component comprising an emollient selected from the group consisting of isododecane, a $C_9$-$C_{12}$ aliphatic hydrocarbon, a $C_9$-$C_{12}$ isoparaffin, mineral oil, isotetracosane, an ester made from a $C_3$-$C_{12}$ alcohol and a $C_3$-$C_{18}$ carboxylic acid, and mixtures thereof; and

(c) a third component selected from the group consisting of a colorant, a sunblock agent, and mixtures thereof.

Preferably, the colorant used in the composition of the invention is selected from the group consisting of titanium dioxide, iron oxide, zinc oxide, mica, and mixtures thereof. Preferred sunblock agents are selected from the group consisting of melanin, melanin protein, submicron particles (i.e., less than 1 micron) of titanium dioxide and iron oxide, octyl methoxycinnamate, benzophenone-3 and mixtures thereof.

We have discovered that the particulate styrene-ethylene-propylene copolymer used in the compositions of the invention provides an effective substrate suitable for transfer and spreading onto the skin in a smooth fashion and for rendering the remaining components of the composition relatively transferproof (i.e., the compositions do not tend to flake-off the skin after application to the skin). The polymers in the compositions of our invention differ from the particulate polymers that are described in the prior art and said to be useful in cosmetic compositions (see, for example, French patent application no. 2367486, PCT application no. WO 88/01164 and European patent application no. 0154831).

The compositions of the invention may also include other ingredients that are suitable for use in cosmetic compositions. Such ingredients include: fragrances, common texture modifiers, such as nylon, polymethylmethacrylate, and agents to control viscosity, such as polyethylene glycol.

The compositions of the invention may be applied in effective amounts to the skin in any suitable manner. The amount applied and the frequency of application will, of course, vary depending upon the composition being applied to the skin and the effect desired.

The particulate styrene-ethylene-propylene copolymer of the present invention preferably comprises from 0.50% to 90% by weight of the composition of the invention. Most preferably, the composition contains from 1.0% to 25% by weight of the copolymer.

Particulate styrene-ethylene-propylene copolymers are well known in the art. For example, suitable styrene-ethylene-propylene copolymers for use in this invention may be obtained, in powdered form, from Shell Chemical Company, Oak Brook, Illinois under the description "Kraton® G Rubber." A particularly preferred material is Kraton® G-1701X. We understand that the G-1701X material is a diblock copolymer having the structure S-EP where "S" denotes a block comprising styrene monomers and "EP" denotes a block comprising ethylene and propylene monomers.

The emollient used in the composition of the invention preferably comprises from 10.0% to 90% by weight of the composition. Most preferably, the emollient comprises from 10% to 70% by weight of the composition.

The amount and identity of emollient(s) used are chosen to achieve the desired property for the intended use of the composition (e.g., for use as a foundation, concealer, blush, etc.). Suitable emollients are isododecane, $C_9$-$C_{12}$ aliphatic hydrocarbons (for example, Permethyl® 99A-D available from Permethyl Corp., Frazer, Pennsylvania), the $C_9$-$C_{12}$ isoparaffins (for example, Isopar® available from Exxon, Houston, Texas), isotetracosane, an ester made from a $C_3$-$C_{12}$ alcohol and a $C_3$-$C_{18}$ carboxylic acid (such as glycerol trioctanoate available as Trivent® OCG from Trivent Chemical Co., Inc., South River, New Jersey; isodecyl isononanoate available as Wickenol® 152 from Wickhen Products, Inc., Huguenot, New York; trioctyl citrate available from Bernel Industries, Engelwood, New Jersey; and isopropyl myristate available as Lexol® IPM from Inolex Chemical Co., Philadelphia, Pennsylvania); and mineral oils (such as the white mineral oils available from Witco Incs., New York, New York). Mixture of emollients may be used in our compositions in order to tailor the properties of the resulting compositions to best fit their intended use.

Colorants used in the present invention are preferably used in amounts such that they comprise 1.0% to 85.0% by weight of the composition and preferably to be in the form of small particles (e.g., particles having an average size of a few microns or less). Most preferably, the colorant comprises 5% to 50% by weight of the composition.

Among suitable colorants are titanium dioxide (such as titanium dioxide #328 available from Whittaker, Clark & Daniels, South Plainfied, New Jersey), iron oxide (such as Cosmetic Yellow-Iron Oxide 7055, available from Whittaker, Clark and Daniels), zinc oxide, boron nitride, colored nylon (for example, Orgasol® available from Lipo, Patterson, New Jersey) and colored polymethylmethacrylate. Any colorant suitable for use in cosmetic compositions and compatible with the other constituents in our composition may be used. Mixtures of colorants may also be used. The selection of a suitable colorant, of course, depends on the color desired and the intended use of the composition (e.g., as a foundation, concealer, blush, etc.).

When a sunblock is used in our composition, it preferably comprises from 0.50% to 90.0% by weight of the composition. Any natural or synthetic sunblock suitable for use in cosmetic compositions may be used. The most preferred natural sunblock for use in the present invention comprises submicron particles of titanium dioxide available from De Gussa, Teterboro, New Jersey. Another natural sunblock is submicron particles of iron oxide. Among suitable synthetic sunblock agents are octyl methoxycinnamate and benzophenone-3.

Techniques commonly used in the cosmetic industry may be used to combine the ingredients of the composition of the present invention. For example, the compositions of our invention may be made by the following steps:

(1) Mixing a styrene-ethylene-propylene copolymer with an emollient by adding the two materials to a vessel and sweepstirring the two materials together at a temperature of 85-90°C for a sufficient period of time to form a transparent, uniform, preliminary dispersion of the copolymer. Preferably the copolymer is added to the vessel in the form of copolymer that had previously been admixed with an emollient.

(2) Cooling the resultant dispersion to room temperature, with continued sidesweeping.

(3) Adding colorant(s) and/or sunblock agent(s) to the preliminary dispersion, with continued sidesweeping until a uniform mixture is obtained. When both colorant(s) and sunblock(s) are added, preferably the soluble colorant(s) and sunblock(s) are added first, followed by adding the nonsoluble sunblock(s) and colorant(s) with sidesweeping until a uniform mixture is obtained.

(4) Adding any additional ingredients that are desired to be in the composition, with sidesweeping until the resulting mixture is uniform.

The following examples are presented for the sole purpose of further illustrating the present invention. Unless otherwise specified, all parts and percentages are by weight.

EXAMPLES

Example 1

A composition suitable for use as foundation and a sunscreen was prepared from the following components in the stated amounts:

| Component | % By Weight |
|---|---|
| 1. Styrene-ethylene-propylene copolymer (Kraton® G-1701X) in the form of a 15% admixture with Isododecane | 20.00 |
| 2. Emollient Isododecane | 17.00 |
| 3. Colorant Titanium Dioxide #328 (Whittaker, Clark & Daniels) Iron Oxide | 15.00<br><br>2.00 |
| 4. Sunscreen Parsol mcx (octyl methoxycinnamate) Spectrasorb UV9 (Benzophenone-3) | 6.00<br>5.00 |
| 5. Texture Modifier Talc | 20.00 |
| 6. Emollient Isotetracosane | 15.00 |

Components 1 and 2 above were combined in accordance with step (1) described above to form a transparent, uniform dispersion. After cooling the resultant dispersion to room temperature as described in step 2 above, components 3, 4 and 5 were added in accordance with steps (3)-(4) above. We also added isotetracosane with these components in order to control the viscosity.

Example 2

A composition suitable for use as a concealer and sunscreen was prepared:

| Component | % By Weight |
|---|---|
| 1. Styrene-ethylene-propylene copolymer (Kraton® G-1701X) in the form of a 15% admixture with Isododecane ..... | 20.00 |
| 2. Emollient Isododecane ........................ | 19.00 |
| 3. Colorant Iron Oxide ........................ | 20.00 |
| 4. Sunscreen Titanium Dioxide #328 (Whittaker, Clark & Daniels) ....... | 3.00 |
| 5. Texture Modifier Talc ................................ | 18.00 |
| 6. Emollient Isotetracosane ..................... | 20.00 |

Components 1 and 2 above were combined in accordance with step (1) described above to form a transparent, uniform dispersion. After cooling the resultant dispersion to room temperature as described in step 2 above, components 3, 4 and 5 were added in accordance with steps (3)-(4) above. We also added isotetracosane with these components in order to control the viscosity.

4

## Claims

1. A cosmetic composition comprising:
   a first component comprising particulate styrene-ethylene-propylene copolymer, wherein the copolymer is a diblock copolymer having the structure S-EP, wherein "S" denotes a block comprising styrene monomers and "EP" denotes a block comprising ethylene and propylene monomers;
   a second component comprising an emollient selected from the group consisting of isododecane, a $C_9$-$C_{12}$ aliphatic hydrocarbon, a $C_9$-$C_{12}$ isoparaffin, mineral oil, isotetracosane, an ester made from a $C_3$-$C_{12}$ alcohol and a $C_3$-$C_{18}$ carboxylic acid, and mixtures thereof; and
   a third component selected from the group consisting of a colorant, a sunblock agent, and mixtures thereof.

2. The cosmetic composition of claim 1, wherein the colorant is selected from the group consisting of titanium dioxide, iron oxide, zinc oxide, mica, and mixtures thereof.

3. The cosmetic composition of claims 1 or 2 wherein the sunblock agent is selected from the group consisting of melanin, melanin protein, titanium dioxide, iron oxide, octyl methoxycinnamate, benzophenone-3, and mixtures thereof.

4. The cosmetic composition of any of claims 1 to 3, wherein the particulate styrene-ethylene-propylene copolymer is present in an amount of about 0.50-90% by weight of the composition.

5. The cosmetic composition of any of claims 1 to 4 wherein the emollient is present in an amount of about 10-90% by weight of the composition.

6. The cosmetic composition of any of claims 1 to 5 wherein the colorant is present in an amount of about 1-85% by weight of the composition.

7. The cosmetic composition of any of claims 1 to 6 wherein the sunscreen is present in an amount of about 0.50-90% by weight of the composition.

8. The cosmetic composition of any of claims 1 to 7 wherein the composition comprises about 1.0-25% by weight of particulate styrene-ethylene-propylene copolymer, about 10-70% by weight of emollient, and about 5.0-50% by weight of colorant.

9. Use of the compositions according to any of claims 1 to 8 as cosmetic compositions to be applied to the skin.

## Patentansprüche

1. Kosmetische Zusammensetzung, umfassend:
   einen ersten Bestandteil, umfassend Styrol-Ethylen-Propylen-Copolymer-Teilchen, wobei das Copolymer ein Diblockcopolymer mit der Struktur S-EP ist, wobei "S" einen Block, umfassend Styrolmonomere bezeichnet, und "EP" einen Block, umfassend Ethylen- und Propylenmonomere bezeichnet;
   einen zweiten Bestandteil, umfassend ein erweichendes Mittel, ausgewählt aus Isododecan, einem $C_9$-$C_{12}$-aliphatischen Kohlenwasserstoff, einem $C_9$-$C_{12}$-Isoparaffin, Mineralöl, Isotetracosan, einem Ester, hergestellt aus einem $C_3$-$C_{12}$-Alkohol und einer $C_3$-$C_{18}$-Carbonsäure, und Gemischen davon; und
   einen dritten Bestandteil, ausgewählt aus einem Farbstoff, einem Sonnenschutzmittel und Gemischen davon.

2. Kosmetische Zusammensetzung nach Anspruch 1, in der der Farbstoff ausgewählt ist aus Titandioxid, Eisenoxid, Zinkoxid, Glimmer und Gemischen davon.

3. Kosmetische Zusammensetzung nach Anspruch 1 oder 2, wobei das Sonnenschutzmittel ausgewählt ist aus Melanin, Melaninprotein, Titandioxid, Eisenoxid, Octylmethoxycinnamat, Benzophenon-3 und Gemischen davon.

4. Kosmetische Zusammensetzung nach einem der Ansprüche 1 bis 3, wobei die Styrol-Ethylen-Propylen-Copolymerteilchen in einer Menge von etwa 0,50 bis 90 Gew.-% der Zusammensetzung vorhanden sind.

5. Kosmetische Zusammensetzung nach einem der Ansprüche 1 bis 4, wobei das erweichende Mittel in einer Menge von etwa 10 bis 90 Gew.-% der Zusammensetzung vorhanden ist.

6. Kosmetische Zusammensetzung nach einem der Ansprüche 1 bis 5, wobei der Farbstoff in einer Menge von etwa 1 bis 85 Gew.-% der Zusammensetzung vorhanden ist.

7. Kosmetische Zusammensetzung nach einem der Ansprüche 1 bis 6, in der der Sonnenschutz in einer Menge von etwa 0,50 bis 90 Gew.-% der Zusammensetzung vorhanden ist.

8. Kosmetische Zusammensetzung nach einem der Ansprüche 1 bis 7, in der die Zusammensetzung etwa 1,0 bis 25 Gew.-% Styrol-Ethylen-Propylen-Copolymerteilchen, etwa 10 bis 70 Gew.-% eines erweichenden Mittels und etwa 5,0 bis 50 Gew.-% Farbstoff umfaßt.

9. Verwendung der Zusammensetzungen nach einem der Ansprüche 1 bis 8 als kosmetische Zusammensetzungen, die auf die Haut aufgetragen werden.

## Revendications

1. Composition cosmétique comprenant :
   un premier composant comprenant un copolymère particulaire de styrène-éthylène-propylène, dans lequel le copolymère est un copolymère de bi-séquence ayant la structure S-EP dans laquelle "S" désigne un bloc ou séquence comprenant des monomères styrène et "EP" désigne un bloc ou séquence comprenant des monomères éthylène et propylène ;
   un second composant comprenant un émollient choisi dans le groupe constitué par l'isododécane, un hydrocarbure aliphatique $C_9$-$C_{12}$, une isoparaffine $C_9$-$C_{12}$, de l'huile minérale, de l'isotétracosane, un ester réalisé à partir d'alcool $C_3$-$C_{12}$ et un acide carboxylique $C_3$-$C_{18}$ et leurs mélanges ; et
   un troisième composant choisi dans le groupe constitué par un colorant, un agent de blocage solaire et leurs mélanges.

2. Composition cosmétique selon la revendication 1, dans laquelle le colorant est choisi à partir du groupe constitué par le dioxyde de titane, l'oxyde de fer, l'oxyde de zinc, le mica et leurs mélanges.

3. Composition cosmétique selon la revendication 1 ou 2, dans laquelle l'agent de blocage solaire est choisi dans le groupe constitué par la mélanine, la protéine mélanine, le dioxyde de titane, l'oxyde de fer, l'octyl-méthoxycinnamate, la benzophénone-3 et leurs mélanges.

4. Composition cosmétique selon l'une quelconque des revendications 1 à 3, dans laquelle le copolymère particulaire de styrène-éthylène-propylène est présent dans une quantité d'environ 0,50-90 % en poids de la composition.

5. Composition cosmétique selon l'une quelconque des revendications 1 à 4, dans laquelle l'émollient est présent dans une quantité d'environ 10-90 % en poids de la composition.

6. Composition cosmétique selon l'une quelconque des revendications 1 à 5, dans laquelle le colorant est présent dans une quantité d'environ 1-85 % en poids de la composition.

7. Composition cosmétique selon l'une quelconque des revendications 1 à 6, dans laquelle l'écran solaire est présent dans une quantité d'environ 0,50-90 % en poids de la composition.

8. Composition cosmétique selon l'une quelconque des revendications 1 à 7, dans laquelle la composition comprend environ 1,0-25 % en poids de copolymère particulaire de styrène-éthyléne-propylène, environ 10-70 % en poids d'émollient et environ 5,0-50 % en poids de colorant.

9. Utilisation des compositions selon l'une quelconque des revendications 1 à 8 comme compositions cosmétiques à appliquer sur la peau.